Europäisches Patentamt

European Patent Office

Office européen des brevets

(11) Veröffentlichungsnummer: **0 057 900**
**B1**

(12) # EUROPÄISCHE PATENTSCHRIFT

(45) Veröffentlichungstag der Patentschrift:
08.05.85

(51) Int. Cl.⁴: **A 23 K 1/16**

(21) Anmeldenummer **82100744.0**

(22) Anmeldetag: **03.02.82**

(54) **Neuer Futterzusatz zur Verbesserung des Wachstums.**

(30) Priorität: **11.02.81 DE 3104850**

(43) Veröffentlichungstag der Anmeldung:
**18.08.82 Patentblatt 82/33**

(45) Bekanntmachung des Hinweises auf die Patenterteilung:
**08.05.85 Patentblatt 85/19**

(84) Benannte Vertragsstaaten:
**AT BE CH DE FR GB IT LI LU NL SE**

(56) Entgegenhaltungen:
**EP - A - 0 002 696**
**US - A - 3 536 712**
**US - A - 4 214 001**

(73) Patentinhaber: **Dr. Karl Thomae GmbH, Postfach 1755, D-7950 Biberach (Riss) (DE)**

(72) Erfinder: **Engelhardt, Günther, Prof., Dr., Unterer Bühl 18, D-7950 Biberach 1 (DE)**
Erfinder: **Pieper, Helmut, Dr., Dipl.-Chem., Kapellenweg 5, D-7950 Biberach 1 (DE)**
Erfinder: **Noll, Klaus, Dr., Dipl.-Chem., Im Schönblick 3, D-7951 Warthausen (DE)**
Erfinder: **Krüger, Gerd, Dr., Dipl.-Chem., Ginsterhalde 30, D-7950 Biberach 1 (DE)**
Erfinder: **Kern, Otto, Dr., Rinderbachstrasse 33, D-6507 Ingelheim (DE)**
Erfinder: **Bomann, Werner, Dr., Königsberger Strasse 21, D-6507 Ingelheim (DE)**

Anmerkung Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

In den US-Patentschriften 3 536 712, 4 063 025, 4 119 710 und 4 214 001 werden u. a. bereits 4-Amino- und 4-Alkoxycarbonylamino-phenyläthanolamine, deren Phenylkern in 3- und/oder 5-Stellung durch Halogenatom, Methyl-, Trifluormethyl-, Cyan- oder Nitrogruppen substituiert sein kann, und deren physiologisch verträgliche Säureadditionssalze beschrieben, welche wertvolle pharmakologische Eigenschaften aufweisen, insbesondere eine $\beta_2$-nimetische Wirkung.

Überraschenderweise wurde nun gefunden, daß bei Verwendung von

1-(4-Äthoxycarbonylamino-3-chlor-5-fluor-phenyl)-2-tert.butylamino-äthanol,
1-(4-Äthoxycarbonylamino-5-brom-3-methyl-phenyl)-2-tert.butylamino-äthanol,
1-(4-Äthoxycarbonylamino-3-chlor-5-trifluormethyl-phenyl)-2-tert.butylamino-äthanol,
1-(4-Äthoxycarbonylamino-3-brom-5-fluor-phenyl)-2-isopropylamino-äthanol,
1-(4-Äthoxycarbonylamino-3-brom-5-fluor-phenyl)-2-tert.butylamino-äthanol,
1-(4-Äthoxycarbonylamino-3-fluor-phenyl)-2-tert.butylamino-äthanol,
1-(4-Äthoxycarbonylamino-3-cyan-5-fluor-phenyl)-2-tert.butylamino-äthanol,
1-(4-Äthoxycarbonylamino-3-nitro-phenyl)-2-tert.butylamino-äthanol,
1-(4-Äthoxycarbonylamino-3-fluor-5-jod-phenyl)-2-cyclopropylamino-äthanol,
1-(3-Cyan-5-fluor-4-isobutyloxycarbonylamino-phenyl)-2-tert.butylamino-äthanol,
1-(3-Fluor-4-isobutyloxycarbonylamino-5-jod-phenyl)-2-cyclopropylamino-äthanol,
1-(4-Äthoxycarbonylamino-3-cyan-phenyl)-2-isopropylamino-äthanol,
1-(4-Äthoxycarbonylamino-3-cyan-phenyl)-2-tert.butylamino-äthanol,
1-(4-Amino-3-fluor-phenyl)2-tert.butylamino-äthanol,
1-(4-Amino-3-chlor-5-fluor-phenyl)-2-isopropylamino-äthanol,
1-(4-Amino-3-chlor-5-fluor-phenyl)-2-cyclopropylamino-äthanol,
1-(4-Amino-3-chlor-5-fluor-phenyl)-2-tert.butylamino-äthanol,
1-(4-Amino-3-chlor-5-fluor-phenyl)-2-tert.pentylamino-äthanol,
1-(4-Amino-3-brom-5-fluor-phenyl)-2-isopropylamino-äthanol,
1-(4-Amino-3-brom-5-flour-phenyl)-2-tert.butylamino-äthanol,
1-(4-Amino-3-brom-5-fluor-phenyl)-2-cyclobutylamino-äthanol,
1-(4-Amino-3-cyan-5-fluor-phenyl)-2-isopropylamino-äthanol,
1-(4-Amino-3-cyan-5-fluor-phenyl)-2-tert.butylamino-äthanol,
1-(4-Amino-3-cyan-5-fluor-phenyl)-2-cyclobutylamino-äthanol,
1-(4-Amino-3-cyan-phenyl)-2-cyclobutylamino-äthanol,
1-(4-Amino-3-cyan-phenyl)-2-tert.pentylamino-äthanol,
1-(4-Amino-3-chlor-5-cyan-phenyl)-2-isopropylamino-äthanol,
1-(4-Amino-3-chlor-5-cyan-phenyl)-2-sec.butylamino-äthanol,
1-(4-Amino-3-chlor-5-cyan-phenyl)-2-tert.pentylamino-äthanol,
1-(4-Amino-3-chlor-5-cyan-phenyl)-2-cyclobutylamino-äthanol,
1-(4-Amino-3-chlor-5-cyan-phenyl)-2-cyclopentylamino-äthanol,
1-(4-Amino-3-brom-5-cyan-phenyl)-2-isopropylamino-äthanol,
1-(4-Amino-3-brom-5-cyan-phenyl)-2-cyclobutylamino-äthanol,
1-(4-Amino-3,5-dicyan-phenyl)-2-tert.butylamino-äthanol,
1-(4-Amino-3-trifluormethyl-phenyl)-2-tert.butylamino-äthanol,
1-(4-Amino-3-trifluormethyl-phenyl)-2-tert.pentylamino-äthanol,
1-(4-Amino-3-chlor-5-trifluormethyl-phenyl)-2-isopropylamino-äthanol,
1-(4-Amino-3-chlor-5-trifluormethyl-phenyl)-2-tert.butylamino-äthanol,
1-(4-Amino-3-chlor-5-trifluormethyl-phenyl)-2-cyclobutylamino-äthanol,
1-(4-Amino-3-chlor-5-trifluormethyl-phenyl)-2-tert.pentylamino-äthanol,
1-(4-Amino-3-brom-5-trifluormethyl-phenyl)-2-isopropylamino-äthanol,
1-(4-Amino-3-chlor-5-nitro-phenyl)-2-tert.butylamino-äthanol,
1-(4-Amino-3-brom-5-nitro-phenyl)-2-tert.butylamino-äthanol und
1-(4-Amino-3-fluor-5-jod-phenyl)-2-cyclopropylamino-äthanol

und von deren physiologisch verträglichen Säureadditionssalzen als Futterzusatz bei der Tierhaltung eine Verbesserung der Futterverwertung, des Futterverbrauchs, der Gewichtszunahme und eine Qualitätsverbesserung der Schlachtkörper, insbesondere jedoch eine Verbesserung des Muskel-/Fett-Verhältnisses erzielt wird.

Hierzu wird erfindungsgemäß eine obige Verbindung in einer Dosierung von 0,001 bis 20 ppm dem Futter beigemischt, so daß eine Tagesdosis von ca. 0,1 bis 20 µg/kg erreicht wird.

Als Masttiere kommen beispielsweise Schweine, Rinder, Geflügel wie Hühner, Enten, Gänse oder Truthühner, Schafe, Kaninchen und Fische in Betracht.

Als besonders geeignetes Futter, dem jeweils eine obige Verbindung oder dessen physiologisch verträgliches Säureadditionssalz in der vorstehend genannten Konzentration beigemischt war, kommt beispielsweise für Schweine Milchaustauschfuttermittel für Ferkel, Alleinfuttermittel für Ferkel (Ferkelaufzuchtfuttermittel), Alleinfuttermittel I für Mastschweine bis etwa 50 kg, Alleinfuttermittel II für Mastschweine von etwa 50 kg an, Alleinfuttermittel für Mastschweine von etwa 35 kg an, Ergänzungsfuttermittel für Ferkel, Ergänzungsfuttermittel I für Mastschweine, Ergänzungsfuttermittel II für Mastschweine, eiweißreiches Ergänzungsfuttermittel für Schweine oder Eiweißkonzentrat für Schweine (Ergänzungsfuttermittel),

für Rinder Milchaustauschfuttermittel für Aufzuchtkälber, Ergänzungsfuttermittel zu Magermilch für Aufzuchtkälber, Ergänzungsfuttermittel für Aufzuchtkälber, Milchaustauschfuttermittel I für Mastkälber, Milchaustauschfuttermittel II für Mastkälber von etwa 80 kg an, energiereiches Ergänzungsfuttermittel zu Magermilch für Mastkälber, Ergänzungsfuttermittel für Mastrinder oder eiweißreiches Ergänzungsfuttermittel für Mastrinder,

für Schafe Milchaustauschfuttermittel für Lämmer oder Alleinfuttermittel für Mastlämmer,

für Geflügel Alleinfuttermittel für Mastgänse, Ergänzungsfuttermittel für Mastgänse, Alleinfuttermittel für Entenküken, Alleinfuttermittel für Jungenten, Alleinfuttermittel für Mastenten, Alleinfuttermittel für Hühnerküken in den ersten Lebenswochen, Alleinfuttermittel für Hühnerküken, Alleinfuttermittel für Junghennen, Alleinfuttermittel I für Masthühnerküken (Broiler), Alleinfuttermittel II für Masthühnerküken (Broiler) von etwa ab der 5. Lebenswoche an, Ergänzungsfuttermittel für Hühnerküken, Alleinfuttermittel für Truthühnerküken, Alleinfuttermittel für Jungtruthühner oder Alleinfuttermittel für Masttruthühner,

für Kaninchen Alleinfuttermittel für Mastkaninchen oder Ergänzungsfuttermittel für Mastkaninchen und

für Fische Alleinfuttermittel für Karpfen oder Alleinfuttermittel für Forellen bzw. entsprechende Beifutter oder Ergänzungsfutter in Betracht.

So beträgt beispielsweise die Konzentration an einer obigen Verbindung oder ihrem physiologisch verträglichem Salz bei Alleinfutter 0,001 bis 10 ppm und bei Ergänzungsfutter 0,002 bis 20 ppm.

Der neue Futterzusatz unterscheidet sich von den bisher bekannten Futterzusätzen insbesondere durch die bevorzugte Wirkung auf die Verbesserung der Qualität des Schlachtkörpers, d. h. durch eine selektive Verbesserung des Muskel/-Fett-Verhältnisses zu Gunsten des Proteinanteils.

Beispielsweise kann diese an Mäusen als dosisabhängige Reduktion des Fettanteils in der Trockensubstanz nachgewiesen werden. Außerdem weist dieser eine geringe Rückstandsproblematik auf, das heißt eine geringe akute Toxizität, keine hemmende Wirkung auf das Bakterienwachstum, somit keine Dysbiose und keine Resistenzbildung bzw. -übertragung.

Beispielsweise wurde die lipolytische Wirkung der Verbindungen

A = 1-(4-Amino-3-cyan-5-fluor-phenyl)-2-tert.butylamino-äthanol-hydrochlorid,
B = 1-(4-Amino-3-cyan-5-fluor-phenyl)-2-isopropylamino-äthanol-hydrochlorid,
C = 1-(4-Amino-3-cyan-5-fluor-phenyl)-2-cyclobutylamino-äthanol-hydrochlorid,
D = 1-(4-Amino-3-chlor-5-cyan-phenyl)-2-isopropylamino-äthanol-hydrochlorid,
E = 1-(4-Amino-3-chlor-5-cyan-phenyl)-2-cyclobutylamino-äthanol-hydrochlorid,
F = 1-(4-Amino-3-chlor-5-cyan-phenyl)-2-cyclopentylamino-äthanol-hydrochlorid und
G = 1-(4-Amino-3-chlor-5-trifluormethylphenyl)-2-cyclobutylamino-äthanol-hydrochlorid

an Kaninchen wie folgt geprüft:

Die zu untersuchenden Substanzen wurden männlichen und weiblichen Kaninchen mit einem durchschnittlichen Gewicht von 2,3 kg, gelöst in physiologischer Kochsalzlösung i. v. injiziert (0,2 mg/kg). Der Fettsäuregehalt des Blutes wurde nach der Methode von Duncombe (Biochem. J. 88, 7 — 10 (1963)) bestimmt. Es wurde die $ED_{50}$, die Dosis, die eine 50%ige Senkung des Fettsäuregehaltes bewirkt, nach Fieler (Quart. J. Pharm. Pharmacol. 17, 117 — 123 (1964)) berechnet.

Die akute Toxizität der zu untersuchenden Substanzen, welche in physiologischer Kochsalzlösung gelöst wurden, wurde an Mäusen mit einem durchschnittlichen Gewicht von 22 g nach i. v. Gabe (0,1 ml/10 g) bestimmt. Es wurde die $LD_{50}$, die Dosis bei welcher 50% der Tiere nach einer Beobachtungszeit von 14 Tagen verstarben, nach der Methode von Litchfield und Wilcoxon (J. Pharmacol. exp. Ther. 96, 99 — 113 (1949)) berechnet.

Die nachfolgende Tabelle enthält die gefunden Werte:

| Verbindung | $ED_{50}$ µg/kg i. v. | $LD_{50}$ mg/kg i. v. |
|---|---|---|
| A | 0,026 | 73,6 |
| B | 0,025 | 66,4 |
| C | 0,047 | —*) |
| D | 0,038 | 81,0 |
| E | 0,083 | 43,9 |
| F | 0,16 | 39,1 |
| G | 0,46 | 36,3 |

*) nicht geprüft

Die nachfolgenden Beispiele sollen die Erfindung näher erläutern:

## Beispiel A

### 1-(4-Äthoxycarbonylamino-3-chlor-5-fluor-phenyl)-2-tert.butylamino-äthanol-hydrochlorid

In eine Lösung von 3,7 g Selendioxid in 30 ml Dioxan und 1 ml Wasser werden bei 60°C unter Rühren 8,7 g 4'-Äthoxycarbonylamino-3'-chlor-5'-fluor-acetophenon portionsweise eingetragen. Anschließend wird 4 Stunden lang auf Rückflußtemperatur erhitzt. Zu der so dargestellten Lösung von 4'-Äthoxycarbonylamino-3'-chlor-5'-fluor-phenylglyoxal werden nach Abkühlen und unter Außenkühlung mit Eis 4,1 ml tert.Butylamin getropft. Nach beendeter Zugabe verdünnt man mit 350 ml Äthanol und filtriert vom Ungelösten ab. Die das rohe 4'-Äthoxycarbonylamino-3'-chlor-5'-fluor-phenyl-glyoxyliden-tert.butylamin enthaltende Lösung wird unter Rühren und Kühlen mit Eis portionsweise mit 5 g Natriumborhydrid und über Nacht bei Raumtemperatur stehen gelassen. Anschließend zerstört man überschüssiges Natriumborhydrid mit Aceton, versetzt mit Wasser und extrahiert mit Chloroform. Die Chloroform-Lösung wird mit Wasser gewaschen, mit Natriumsulfat getrocknet, nach Zusatz von Aktivkohle kurz aufgekocht, filtriert und im Vakuum zur Trockne eingedampft. Der feste Rückstand von 1-(4-Äthoxycarbonylamino-3-chlor-5-fluor-phenyl)-2-tert.butylamino-äthanol wird in Isopropanol aufgenommen und mit ätherischer Salzsäure bis pH 4 angesäuert. Nach Zugabe von Äther tritt Kristallisation ein. Die Kristalle weden abgesaugt und mit Äther gewaschen. Schmelzpunkt: 192—193°C.

## Beispiel B

### 1-(4-Äthoxycarbonylamino-5-brom-3-methyl-phenyl)-2-tert.butylamino-äthanol

12 g 4'-Äthoxycarbonylamino-5'-brom-3'-methyl-acetophenon werden in 200 ml Chloroform gelöst und zum Sieden erhitzt. In die siedende Lösung tropft man 6,5 g Brom, das unter Bromwasserstoffentwicklung schnell verbraucht wird. Danach wird das jetzt in der Lösung vorhandene 4'-Äthoxycarbonylamino-5',2-dibrom-3'-methyl-acetophenon durch Zugabe von 14,6 g tert.Butylamin und 30minütiges Kochen in 4'-Äthoxycarbonylamino-5'-brom-3'-methyl-2-tert.butylamino-acetophenon überführt. Man engt im Vakuum zur Trockne ein, nimmt den Rückstand in 50 ml Methanol und 20 ml Wasser auf und reduziert die Ketogruppe durch Zugabe einer Lösung von 3,8 g Natriumborhydrid in 20 ml Wasser, wobei gleichzeitig der pH-Wert des Reaktionsgemisches durch Zugabe von verdünnter Salzsäure zwischen 6 und 8 gehalten wird. Nach Beendigung der Reduktion wird das Methanol im Vakuum abdestilliert, der Rückstand mit Wasser verdünnt, mit Ammoniak deutlich alkalisch gestellt und mit Chloroform extrahiert. Die organische Phase wird abgetrennt, getrocknet und zur Trockne eingeengt. Der Rückstand wird über eine Kieselgelsäule gereinigt (Elutionsmittel: Chloroform/Methanol 2 : 1), das Eluat eingeengt, der Rückstand in Äthanol gelöst und mit ätherischer Salzsäure versetzt. Man erhält 1-(4-Äthoxy-carbonylamino-5-brom-3-methylphenyl)-2-tert.butylamino-äthanol-hydrochlorid. Schmelzpunkt: 212—214°C (Zers.).

Analog Beispiel A oder B werden folgende Verbindungen hergestellt:

4

1-(4-Äthoxycarbonylamino-3-chlor-5-trifluormethyl-phenyl)-2-tert.butylamino-äthanol
Schmelzpunkt: 168—170° C (Zers.).
1-(4-Äthoxycarbonylamino-3-brom-5-fluor-phenyl)-2-isopropylamino-äthanol-hydrochlorid
Schmelzpunkt: 180—182° C
1-(4-Äthoxycarbonylamino-3-brom-5-fluor-phenyl)-2-tert.butylamino-äthanol-hydrochlorid
Schmelzpunkt: 197—198° C (Zers.)
1-(4-Äthoxycarbonylamino-3-fluor-phenyl)-2-tert.butylamino-äthanol-hydrochlorid
Schmelzpunkt: 235—236° C
1-(4-Äthoxycarbonylamino-3-cyan-5-fluor-phenyl)-2-tert.butylamino-äthanol-hydrochlorid
Schmelzpunkt: 198—200° C (Zers.)
1-(4-Äthoxycarbonylamino-3-nitro-phenyl)-2-tert.butylamino-äthanol-hydrochlorid
Schmelzpunkt: 189—190° C (Ters.)
1-(4-Äthoxycarbonylamino-3-fluor-5-jod-phenyl)-2-cyclopropylamin-äthanol
Schmelzpunkt: 127—130° C
1-(3-Cyan-5-fluor-4-isobutyloxycarbonylamino-phenyl)-2-tert.butylamino-äthanol-hydrochlorid
(Das überschüssige Natriumborhydrid wurde mit verdünnter Salzsäure zerstört)
Schmelzpunkt: 189—191° C
1-(3-Fluor-4-isobutyloxycarbonylamino-5-jod-phenyl)-2-cycloproylamino-äthanol
(Das überschüssige Natriumborhydrid wurde mit verdünnter Salzsäure zerstört)
Schmelzpunkt: 126—128° C
1-(4-Äthoxycarbonylamino-3-cyan-phenyl)-2-isopropylamino-äthanol
Schmelzpunkt: 112—115° C
1-(4-Äthoxycarbonylamino-3-phenyl)-2-tert.butylamino-äthanol
Schmelzpunkt: 78—82° C
1-(4-Amino-3-fluor-phenyl)-2-tert.butylamino-äthanol-hydrochlorid
Schmelzpunkt: 196—197° C (Zers.)
1-(4-Amino-3-chlor-5-fluor-phenyl)-2-isopropylamino-äthanol-hydrochlorid
Schmelzpunkt: 152—154° C (Zers.)
1-(4-Amino-3-chlor-5-fluor-phenyl)-2-cyclopropylamino-äthanol-hydrochlorid
Schmelzpunkt: 175—177° C (Zers.)
1-(4-Amino-3-chlor-5-fluor-phenyl)-2-tert.butylamino-äthanol-hydrochlorid
Schmelzpunkt: 206—208° C (Zers.)
1-(4-Amino-3-chlor-5-fluor-phenyl)-2-tert.pentylamino-äthanol-hydrochlorid
Schmelzpunkt: 187—188° C (Zers.)
1-(4-Amino-3-brom-5-fluor-phenyl)-2-isopropylamino-äthanol-hydrochlorid
Schmelzpunkt: 171—173° C (Zers.)
1-(4-Amino-3-brom-5-fluor-phenyl)-2-tert.butylamino-äthanol-hydrochlorid
Schmelzpunkt: 207—208° C (Zers.)
1-(4-Amino-3-brom-fluor-phenyl)-2-cyclobutylamino-äthanol-hydrochlorid
Schmelzpunkt: 164—166° C (Zers.)
1-(4-Amino-3-cyan-5-fluor-phenyl)-2-isopropylamino-äthanol-hydrochlorid
Schmelzpunkt: 182—184° C (Zers.)
1-(4-Amino-3-cyan-5-fluor-phenyl)-2-tert.butylamino-äthanol-hydrochlorid
Schmelzpunkt: 242—243° C (Zers.)
1-(4-Amino-3-cyan-phenyl)-2-cyclobutylamino-äthanol-hydrobromid
Schmelzpunkt: ab 193° C (Zers.)
1-(4-Amino-3-cyan-phenyl)-2-tert.pentylamino-äthanol
Schmelzpunkt: 143° C
1-(4-Amino-3-chlor-5-cyan-phenyl)-2-sec.butylamino-äthanol-dihydrochlorid
Schmelzpunkt: 190—191° C
1-(4-Amino-3-chlor-5-cyan-phenyl)-2-tert.pentylamino-äthanol-hydrochlorid
Schmelzpunkt: 218—220° C (Zers.)
1-(4-Amino-3-chlor-5-cyan-phenyl)-2-cyclopentylamino-äthanol-hydrochlorid
Schmelzpunkt: 138—144° C
1-(4-Amino-3-brom-5-cyan-phenyl)-2-isopropylamino-äthanol-hydrochlorid
Schmelzpunkt: 186—189° C
1-(4-Amino-3-brom-5-cyan-phenyl)-2-cyclobutylamino-äthanol-hydrochlorid
Schmelzpunkt: 215—216° C (Zers.)
1-(4-Amino-3,5-dicyan-phenyl)-2-tert.butylamino-äthanol-hydrochlorid
Schmelzpunkt: 251—253° C (Zers.)
1-(4-Amino-3-trifluormethyl-phenyl)-2-tert.butylamino-äthanol-hydrochlorid
Schmelzpunkt: 172—174° C (Zers.)
1-(4-Amino-3-trifluormethyl-phenyl)-2-tert.pentylamino-äthanol-hydrobromid
Schmelzpunkt: 174—175° C (Zers.)
1-(4-Amino-3-chlor-5-trifluormethyl-phenyl)-2-isopropylamino-äthanol Schmelzpunkt: 104—106°C
1-(4-Amino-3-chlor-5-trifluormethyl-phenyl)-2-tert.butylamino-äthanol-hydrochlorid
Schmelzpunkt: 205—207°C (Zers.)
1-(4-Amino-3-chlor-5-trifluormethyl-phenyl)-2-cyclobutylamino-äthanol-hydrochlorid
Schmelzpunkt: 177—178°C
1-(4-Amino-3-chlor-5-triflourmethyl-phenyl)-2-tert.pentylamino-äthanol-hydrochlorid
Schmelzpunkt: 176—178°C (Zers.)
1-(4-Amino-3-brom-5-trifluormethyl-phenyl)-2-isopropylamino-äthanol-hydrochlorid
Schmelzpunkt: 177—179°C (Zers.)
1-(4-Amino-3-chlor-5-nitro-phenyl)-2-tert.butylamino-äthanol
Schmelzpunkt: 148—149°C
1-(4-Amino-3-brom-5-nitro-phenyl)-2-tert.butylamino-äthanol
Schmelzpunkt: 151—152°C
1-(4-Amino-3-fluor-5-jod-phenyl)-2-cyclopropylamino-äthanol-hydrochlorid
Schmelzpunkt: 199—201°C (Zers.)

## Beispiel 1

### Alleinfuttermittel für Ferkel (Ferkelaufzuchtfuttermittel)

| a) | Rohprotein | min. 17% (einschließlich min. 0,85% Lysin) |
|---|---|---|
| | Rohfett | max. 7% |
| | Rohfaser | 3,5—6% |
| | Rohasche | max. 7% |
| | Stärke | min. 33% |
| | Stärke/Zucker/Rohfett | min. 46% |
| | Calcium | min. 0,8% |
| | Phosphor | min. 0,6% |
| | Natrium | min. 0,2% |
| b) | Eisen | min. 100 mg |
| | Kupfer | min. 20 mg |
| | Mangan | min. 30 mg |
| | Zink | min. 70 mg |
| | Vitamin A | min. 8000 I.E. |
| | Vitamin D | min. 1000 I.E. |
| | 1-(4-Amino-3-chlor-5-trifluor-methylphenyl)-2-tert.butylamino-äthanol-hydrochlorid | 0,001—10 ppm |

## Beispiel 2

### Alleinfuttermittel I für Mastschweine bis etwa 50 kg

| a) | Rohprotein | min. 16% (einschließlich min. 0,75% Lysin) |
|---|---|---|
| | Rohfett | max. 8% |
| | Rohfaser | max. 6% |
| | Rohasche | max. 7% |
| | Stärke | min. 33% |
| | Stärke/Zucker/Rohfett | min. 47% |
| | Calcium | min. 0,7% |
| | Phosphor | min. 0,5% |
| | Natrium | min. 0,15% |
| b) | Kupfer | min. 20 mg |
| | Zink | min. 50 mg |
| | Vitamin A | min. 4000 I.E. |
| | Vitamin D | min. 500 I.E. |
| | 1-(4-Äthoxycarbonylamino-3-cyan-5-fluorphenyl)-2-tert.butyl-amino-äthanol-hydrochlorid | 0,001—10 ppm |

# 0 057 900

### Beispiel 3

#### Ergänzungsfuttermittel I für Mastschweine

a) Rohprotein      22—26% (einschließlich min. 1,2% Lysin)
Rohfett      max. 12%
Rohfaser      max. 7%
Rohasche      max. 13%
Calcium      min. 2,2%
Phosphor      min. 0,7%
Natrium      min. 0,25%
b) Kupfer      min. 40 mg
Zink      min. 200 mg
Vitamin A      min. 8000 I.E.
Vitamin D      min. 1000 I.E.
1-(4-Amino-3-chlor-5-trifluor-
methyl-phenyl)-2-tert.butyl-
amino-äthanol-hydrochlorid      0,002—20 ppm

### Beispiel 4

#### Milchaustauschfuttermittel I für Mastkälber

a) Rohprotein      min. 32% (einschließlich min. 1,75% Lysin)
Rohfett      12 bis 30%
Rohfaser      max. 1,5%
Rohasche      max. 10%
Calcium      min. 0,9%
Phosphor      min. 0,7%
Magnesium      min. 0,13%
Natrium      0,25 bis 0,7%
Milchpulver      min. 50% (einschließlich max. 20% Buttermilchpulver)
b) Eisen      min. 30 mg
Kupfer      4 bis 15 mg
Vitamin A      min. 10 000 I.E.
Vitamin D      min. 1250 I.E.
Vitamin E      min. 20 mg
1-(4-Amino-3-chlor-5-trifluor-
methylphenyl)-2-tert.butylamino-
äthanol-hydrochlorid      0,002—20 ppm

### Beispiel 5

#### Ergänzungsfuttermittel für Mastrinder

a) Rohprotein      15 bis 25% (einschließlich max. 6% Rohprotein
aus npn-Verbindungen)
Rohfett      max. 10%
Rohfaser      max. 12%
Rohasche      max. 9%
Calcium      0,8 bis 1,2%
1-(4-Äthoxycarbonylamino-3-cyan-
5-fluorphenyl)-2-tert.butyl-
amino-äthanol-hydrochlorid      0,002—20 ppm

7

### Beispiel 6

#### Alleinfuttermittel für Mastlämmer

a) Rohprotein min. 16%
Rohfaser max. 8%
Rohasche max. 9%
Calcium min. 1%
Phosphor min. 0,5%
Ca : P-Verhältnis nicht unter 2 : 1
b) Kupfer max. 12 mg
Vitamin A min. 10 000 I.E.
Vitamin $D_3$ min. 1250 I.E.
Vitamin E min. 12 mg
1-(4-Amino-3-chlor-5-trifluor-
methylphenyl)-2-tert.butyl-
amino-äthanol-hydrochlorid 0,001 — 10 ppm

### Beispiel 7

#### Alleinfuttermittel I für Masthühnerküken (Broiler)

a) Rohprotein min. 22% (einschließlich min. 0,45% Methionin)
Zucker max. 12%
Stärke/Zucker/Rohfett min. 51%
Calcium 0,7 bis 1,2%
Phosphor min. 0,6%
Natrium 0,12 bis 0,3%
b) Mangan min. 50 mg
Zink min. 50 mg
Vitamin A min. 6000 I.E.
Vitamin $D_3$ min. 750 I.E.
Riboflavin (Vitamin $B_2$) min. 4 mg
Vitamin $B_{12}$ min. 10 mg
1-(4-Amino-3-chlor-5-trifluor-
methyl-phenyl)-2-tert.butyl-
amino-äthanol-hydrochlorid 0,001 — 10 ppm

### Beispiel 8

#### Alleinfuttermittel für Mastkaninchen

Rohprotein 10 — 30%
Rohfaser 10 — 20%
Calcium 0,5 — 1,5%
Phosphor min. 0,5%
1-(4-Amino-3-chlor-5-trifluor-
methyl-phenyl)-2-tert.butyl-
amino-äthanol-hydrochlorid 0,001 — 20 ppm

### Beispiel 9

#### Alleinfuttermittel für Karpfen

Rohprotein 10 — 40%
Rohfett 10 — 20%
Rohfaser max. 10%
1-(4-Amino-3-chlor-5-trifluor-
methyl-phenyl)-2-tert.butyl-
amino-äthanol-hydrochlorid 0,001 — 20 ppm

**0 057 900**

**Patentansprüche**

1. Verwendung von

1-(4-Äthoxycarbonylamino-3-chlor-5-fluor-phenyl)-2-tert.butylamino-äthanol,
1-(4-Äthoxycarbonylamino-5-brom-3-methyl-phenyl)-2-tert.butylamino-äthanol,
1-(4-Äthoxycarbonylamino-3-chlor-5-trifluormethyl-phenyl)-2-tert.butylamino-äthanol,
1-(4-Äthoxycarbonylamino-3-brom-5-fluor-phenyl)-2-isopropylamino-äthanol,
1-(4-Äthoxycarbonylamino-3-brom-5-fluor-phenyl)-2-tert.butylamino-äthanol,
1-(4-Äthoxycarbonylamino-3-fluor-phenyl)-2-tert.butylamino-äthanol,
1-(4-Äthoxycarbonylamino-3-cyan-5-fluor-phenyl)-2-tert.butylamino-äthanol,
1-(4-Äthoxycarbonylamino-3-nitro-phenyl)-2-tert.butylamino-äthanol,
1-(4-Äthoxycarbonylamino-3-fluor-5-jod-phenyl)-2-cyclopropylamino-äthanol,
1-(3-Cyan-5-fluor-4-isobutyloxycarbonylamino-phenyl)-2-tert.butylamino-äthanol,
1-(3-Fluor-4-isobutyloxycarbonylamino-5-jod-phenyl)-2-cyclopropylamino-äthanol,
1-(4-Äthoxycarbonylamino-3-cyan-phenyl)-2-isopropylamino-äthanol,
1-(4-Äthoxycarbonylamino-3-cyan-phenyl)-2-tert.butylamino-äthanol,
1-(4-Amino-3-fluor-phenyl)-2-tert.butylamino-äthanol,
1-(4-Amino-3-chlor-5-fluor-phenyl)-2-isopropylamino-äthanol,
1-(4-Amino-3-chlor-5-fluor-phenyl)-2-cyclopropylamino-äthanol,
1-(4-Amino-3-chlor-5-fluor-phenyl)-2-tert.butylamino-äthanol,
1-(4-Amino-3-chlor-5-fluor-phenyl)-2-tert.pentylamino-äthanol,
1-(4-Amino-3-brom-5-fluor-phenyl)-2-isopropylamino-äthanol,
1-(4-Amino-3-brom-5-fluor-phenyl)-2-tert.butylamino-äthanol,
1-(4-Amino-3-brom-5-fluor-phenyl)-2-cyclobutylamino-äthanol,
1-(4-Amino-3-cyan-5-fluor-phenyl)-2-isopropylamino-äthanol,
1-(4-Amino-3-cyan-5-fluor-phenyl)-2-tert.butylamino-äthanol,
1-(4-Amino-3-cyan-5-fluor-phenyl)-2-cyclobutylamino-äthanol,
1-(4-Amino-3-cyan-phenyl)-2-cyclobutylamino-äthanol,
1-(4-Amino-3-cyan-phenyl)-2-tert.pentylamino-äthanol,
1-(4-Amino-3-chlor-5-cyan-phenyl)-2-isopropylamino-äthanol,
1-(4-Amino-3-chlor-5-cyan-phenyl)-2-sec.butylamino-äthanol,
1-(4-Amino-3-chlor-5-cyan-phenyl)-2-tert.pentylamino-äthanol,
1-(4-Amino-3-chlor-5-cyan-phenyl)-2-cyclobutylamino-äthanol,
1-(4-Amino-3-chlor-5-cyan-phenyl)-2-cyclopentylamino-äthanol,
1-(4-Amino-3-brom-5-cyan-phenyl)-2-isopropylamino-äthanol,
1-(4-Amino-3-brom-5-cyan-phenyl)-2-cyclobutylamino-äthanol,
1-(4-Amino-3,5-dicyan-phenyl)-2-tert.butylamino-äthanol,
1-(4-Amino-3-trifluormethyl-phenyl)-2-tert.butylamino-äthanol,
1-(4-Amino-3-trifluormethyl-phenyl)-2-tert.pentylamino-äthanol,
1-(4-Amino-3-chlor-5-trifluormethyl-phenyl)-2-isopropylamino-äthanol,
1-(4-Amino-3-chlor-5-trifluormethyl-phenyl)-2-tert.butylamino-äthanol,
1-(4-Amino-3-chlor-5-trifluormethyl-phenyl)-2-cyclobutylamino-äthanol,
1-(4-Amino-3-chlor-5-trifluormethyl-phenyl)-2-tert.pentylamino-äthanol,
1-(4-Amino-3-brom-5-trifluormethyl-phenyl)-2-isopropylamino-äthanol,
1-(4-Amino-3-chlor-5-nitro-phenyl)-2-tert.butylamino-äthanol,
1-(4-Amino-3-brom-5-nitro-phenyl)-2-tert.butylamino-äthanol und
1-(4-Amino-3-fluor-5-jod-phenyl)-2-cyclopropylamino-äthanol

sowie deren Säureadditionssalze als Futtermittelzusatz zur Verbesserung des Wachstums von Tieren.

2. Verwendung von 1-(4-Amino-3-chlor-5-trifluormethyl-phenyl)-2-tert.butylamino-äthanol und dessen Säureadditionssalze als Futtermittelzusatz zur Verbesserung des Wachstums von Tieren.

3. Verwendung von 1-(4-Äthoxycarbonylamino-3-cyan-5-fluor-phenyl)-2-tert.-butylamino-äthanol und dessen Säureadditionssalze als Futtermittelzusatz zur Verbesserung des Wachstums von Tieren.

4. Verwendung einer Verbindung gemäß den Ansprüchen 1 bis 3 als Futtermittelzusatz zur Verbesserung der Futterverwertung, zur Verbesserung der Gewichtszunahme und zur Qualitätsverbesserung der Schlachtkörper von Tieren.

5. Verwendung einer Verbindung gemäß den Ansprüchen 1 bis 3 als Futtermittelzusatz zur Steigerung der Gewichtszunahme und zur Qualitätssteigerung der Schlachtkörper von Tieren.

6. Verwendung einer Verbindung gemäß den Ansprüchen 1 bis 3 als Futtermittelzusatz in einer Konzentration von 0,001 bis 20 ppm.

7. Futtermittel, enthaltend eine Verbindung gemäß den Ansprüchen 1 bis 3 in einer Konzentration von 0,001 bis 20 ppm.

9

8. Verfahren zur Herstellung eines neuen Futtermittels, dadurch gekennzeichnet, daß ein übliches Futtermittel mit einer Verbindung gemäß den Ansprüchen 1 bis 3 bis zu einer Konzentration von 0,001 bis 20 ppm vermischt wird.

**Claims**

1. Use of

1-(4-Ethoxycarbonylamino-3-chloro-5-fluoro-phenyl)-2-tert.butylamino-ethanol,
1-(4-Ethoxycarbonylamino-5-bromo-3-methyl-phenyl)-2-tert.butylamino-ethanol,
1-(4-Ethoxycarbonylamino-3-chloro-5-trifluoromethyl-phenyl)-2-tert.butylamino-ethanol,
1-(4-Ethoxycarbonylamino-3-bromo-5-fluoro-phenyl)-2-isopropylamino-ethanol,
1-(4-Ethoxycarbonylamino-3-bromo-5-fluoro-phenyl)-2-tert.butylamino-ethanol,
1-(4-Ethoxycarbonylamino-3-fluoro-phenyl)-2-tert.butylamino-ethanol,
1-(4-Ethoxycarbonylamino-3-cyano-5-fluoro-phenyl)-2-tert.butylamino-ethanol,
1-(4-Ethoxycarbonylamino-3-nitro-phenyl)-2-tert.butylamino-ethanol,
1-(4-Ethoxycarbonylamino-3-fluoro-5-iodo-phenyl)-2-cyclopropylamino-ethanol,
1-(3-Cyano-5-fluoro-4-isobutyloxycarbonylamino-phenyl)-2-tert.butylamino-ethanol,
1-(3-Fluoro-4-isobutyloxycarbonylamino-5-iodo-phenyl)-2-cyclopropylamino-ethanol,
1-(4-Ethoxycarbonylamino-3-cyano-phenyl)-2-isopropylamino-ethanol,
1-(4-Ethoxycarbonylamino-3-cyano-phenyl)-2-tert.butylamino-ethanol,
1-(4-Amino-3-fluoro-phenyl)-2-tert.butylamino-ethanol,
1-(4-Amino-3-chloro-5-fluoro-phenyl)-2-isopropylamino-ethanol,
1-(4-Amino-3-chloro-5-fluoro-phenyl)-2-cyclopropylamino-ethanol,
1-(4-Amino-3-chloro-5-fluoro-phenyl)-2-tert.butylamino-ethanol,
1-(4-Amino-3-chloro-5-fluoro-phenyl)-2-tert.pentylamino-ethanol,
1-(4-Amino-3-bromo-5-fluoro-phenyl)-2-isopropylamino-ethanol,
1-(4-Amino-3-bromo-5-fluoro-phenyl)-2-tert.butylamino-ethanol,
1-(4-Amino-3-bromo-5-fluoro-phenyl)-2-cyclobutylamino-ethanol,
1-(4-Amino-3-cyano-5-fluoro-phenyl)-2-isopropylamino-ethanol,
1-(4-Amino-3-cyano-5-fluoro-phenyl)-2-tert.butylamino-ethanol,
1-(4-Amino-3-cyano-5-fluoro-phenyl)-2-cyclobutylamino-ethanol,
1-(4-Amino-3-cyano-phenyl)-2-cyclobutylamino-ethanol,
1-(4-Amino-3-cyano-phenyl)-2-tert.pentylamino-ethanol,
1-(4-Amino-3-chloro-5-cyano-phenyl)-2-isopropylamino-ethanol,
1-(4-Amino-3-chloro-5-cyano-phenyl)-2-sec.butylamino-ethanol,
1-(4-Amino-3-chloro-5-cyano-phenyl)-2-tert.pentylamino-ethanol,
1-(4-Amino-3-chloro-5-cyano-phenyl)-2-cyclobutylamino-ethanol,
1-(4-Amino-3-chloro-5-cyano-phenyl)-2-cyclopentylamino-ethanol,
1-(4-Amino-3-bromo-5-cyano-phenyl)-2-isopropylamino-ethanol,
1-(4-Amino-3-bromo-5-cyano-phenyl)-2-cyclobutylamino-ethanol,
1-(4-Amino-3,5-dicyano-phenyl)-2-tert.butylamino-ethanol,
1-(4-Amino-3-trifluoromethyl-phenyl)-2-tert.butylamino-ethanol,
1-(4-Amino-3-trifluoromethyl-phenyl)-2-tert.pentylamino-ethanol,
1-(4-Amino-3-chloro-5-trifluoromethyl-phenyl)-2-isopropylamino-ethanol,
1-(4-Amino-3-chloro-5-trifluoromethyl-phenyl)-2-tert.butylamino-ethanol,
1-(4-Amino-3-chloro-5-trifluoromethyl-phenyl)-2-cyclobutylamino-ethanol,
1-(4-Amino-3-chloro-5-trifluoromethyl-phenyl)-2-tert.pentylamino-ethanol,
1-(4-Amino-3-bromo-5-trifluoromethyl-phenyl)-2-isopropylamino-ethanol,
1-(4-Amino-3-chloro-5-nitro-phenyl)-2-tert.butylamino-ethanol,
1-(4-Amino-3-bromo-5-nitro-phenyl)-2-tert.butylamino-ethanol and
1-(4-Amino-3-fluoro-5-iodo-phenyl)-2-cyclopropylamino-ethanol

and the acid addition salts thereof as feed additives for improving the growth of animals.

2. Use of 1-(4-Amino-3-chloro-5-trifluoromethylphenyl)-2-tert.butylamino-ethanol and the acid addition salts thereof as a feed additive for improving the growth of animals.

3. Use of 1-(4-Ethoxycarbonylamino-3-cyano-5-fluoro-phenyl)-2-tert.butylamino-ethanol and the acid addition salts thereof as a feed additive for improving the growh of animals.

4. Use of a compound as claimed in claims 1 to 3 as a feed additive for improving utilisation of feed, for improving the weight again and for improving the quality of the slaughtered caracasses of animals.

5. Use of a compound as claimed in claims 1 to 3 as a feed additive for increasing the weight gain and improving the quality of the slaughtered carcasses of animals.

6. Use of a compound as claimed in claims 1 to 3 as a feed additive in a concentration of from 0.001 to 20 ppm.

**0 057 900**

7. Foodstuff containing a compound as claimed in claims 1 to 3 in a concentration of from 0.001 to 20 ppm.

8. Process for producing a new foodstuff, characterised in that a conventional foodstuff is mixed with a compound as claimed in claims 1 to 3 to give a concentration of from 0.001 to 20 ppm.

**Revendications**

1. Utilisation du:

1-(4-éthoxycarbonylamino-3-chloro-5-fluoro-phényl)-2-tert.butylamino-éthanol,
1-(4-éthoxycarbonylamino-5-bromo-3-méthyl-phényl)-2-tert.butylamino-éthanol,
1-(4-éthoxycarbonylamino-3-chloro-5-trifluorométhyl-phényl)-2-tert.butylamino-éthanol,
1-(4-éthoxycarbonylamino-3-bromo-5-fluoro-phényl)-2-isopropylamino-éthanol,
1-(4-éthoxycarbonylamino-3-bromo-5-fluoro-phényl)-2-tert.butylamino-éthanol,
1-(4-éthoxycarbonylamino-3-fluoro-phényl)-2-tert.butylamino-éthanol,
1-(4-éthoxycarbonylamino-3-cyano-5-fluoro-phényl)-2-tert.butylamino-éthanol,
1-(4-éthoxycarbonylamino-3-nitro-phényl)-2-tert.butylamino-éthanol,
1-(4-éthoxycarbonylamino-3-fluoro-5-iodo-phényl)-2-cyclopropylamino-éthanol,
1-(3-cyano-5-fluoro-4-isobutyloxycarbonylamino-phényl)-2-tert.butylamino-éthanol,
1-(3-fluoro-4-isobutyloxycarbonylamino-5-iodo-phényl)-2-cyclopropylamino-éthanol,
1-(4-éthoxycarbonylamino-3-cyano-phényl)-2-isopropylamino-éthanol,
1-(4-éthoxycarbonylamino-3-cyano-phényl)-2-tert.butylamino-éthanol,
1-(4-amino-3-fluoro-phényl)-2-tert.butylamino-éthanol,
1-(4-amino-3-chloro-5-fluoro-phényl)-2-isopropylamino-éthanol,
1-(4-amino-3-chloro-5-fluoro-phényl)-2-cyclopropylamino-éthanol,
1-(4-amino-3-chloro-5-fluoro-phényl)-2-tert.butylamino-éthanol,
1-(4-amino-3-chloro-5-fluoro-phényl)-2-tert.pentylamino-éthanol,
1-(4-amino-3-bromo-5-fluoro-phényl)-2-isopropylamino-éthanol,
1-(4-amino-3-bromo-5-fluoro-phényl)-2-tert.butylamino-éthanol,
1-(4-amino-3-bromo-5-fluoro-phényl)-2-cyclobutylamino-éthanol,
1-(4-amino-3-cyano-5-fluoro-phényl)-2-isopropylamino-éthanol,
1-(4-amino-3-cyano-5-fluoro-phényl)-2-tert.butylamino-éthanol,
1-(4-amino-3-cyano-5-fluoro-phényl)-2-cyclobutylamino-éthanol,
1-(4-amino-3-cyano-phényl)-2-cyclobutylamino-éthanol,
1-(4-amino-3-cyano-phényl)-2-tert.pentylamino-éthanol,
1-(4-amino-3-chloro-5-cyano-phényl)-2-isopropylamino-éthanol,
1-(4-amino-3-chloro-5-cyano-phényl)-2-sec.butylamino-éthanol,
1-(4-amino-3-chloro-5-cyano-phényl)-2-tert.pentylamino-éthanol,
1-(4-amino-3-chloro-5-cyano-phényl)-2-cyclobutylamino-éthanol,
1-(4-amino-3-chloro-5-cyano-phényl)-2-cyclopentylamino-éthanol,
1-(4-amino-3-bromo-5-cyano-phényl)-2-isopropylamino-éthanol,
1-(4-amino-3-bromo-5-cyano-phényl)-2-cyclobutylamino-éthanol,
1-(4-amino-3,5-dicyano-phényl)-2-tert.butylamino-éthanol,
1-(4-amino-3-trifluorométhyl-phényl)-2-tert.butylamino-éthanol,
1-(4-amino-3-trifluorométhyl-phényl)-2-tert.pentylamino-éthanol,
1-(4-amino-3-chloro-5-trifluorométhyl-phényl)-2-isopropylamino-éthanol,
1-(4-amino-3-chloro-5-trifluorométhyl-phényl)-2-tert.butylamino-éthanol,
1-(4-amino-3-chloro-5-trifluorométhyl-phényl)-2-cyclobutylamino-éthanol,
1-(4-amino-3-chloro-5-trifluorométhyl-phényl)-2-tert.pentylamino-éthanol,
1-(4-amino-3-bromo-5-trifluorométhyl-phényl)-2-isopropylamino-éthanol,
1-(4-amino-3-chloro-5-nitro-phényl)-2-tert.butylamino-éthanol,
1-(4-amino-3-bromo-5-nitro-phényl)-2-tert.butylamino-éthanol, et
1-(4-amino-3-fluoro-5-iodo-phényl)-2-cyclopropylamino-éthanol,

ainsi que de leurs sels d'addition d'cides en tant qu'additif pour l'alimentation animale, pour améliorer la croissance des animaux.

2. Utilisation du 1-(4-amino-3-chloro-5-trifluorométhyl-phényl)-2-tert.butylamino-éthanol et de ses sels d'addition d'acides en tant qu'additif pour l'alimentation animale, pour améliorer la croissance des animaux.

3. Utilisation du 1-(4-éthoxycarbonylamino-3-cyano-5-fluoro-phényl)-2-tert.butylamino-éthanol et de ses sels d'addition d'acides en tant qu'additif pour l'alimentation animale, pour améliorer la croissance des animaux.

4. Utilisation d'un composé selon les revendications 1 à 3 en tant qu'additif pour l'alimentation animale pour améliorer la mise à profit de l'alimentation, pour améliorer la croissance du poids et pour

11

améliorer la qualité du corps à l'abattage (carcasse) des animaux.

5. Utilisation d'un composé selon les revendications 1 à 3 en tant qu'additif pour l'alimentation animale pour augmenter la croissance du poids et pour augmenter la qualité du corps à l'abattage (carcasse) des animaux.

6. Utilisation d'un composé selon les revendications 1 à 3 en tant qu'additif pour l'alimentation animale en une concentration de 0,001 à 20 ppm.

7. Aliment pour les animaux contenant un composés selon les revendications 1 à 3 en une concentration de 0,001 à 20 ppm.

8. Procédé pour la préparation d'un nouvel aliment pour animaux, caractérisé en ce qu'on mélange un aliment usuel pour animaux avec un composé selon les revendications 1 à 3 jusqu'à une concentration de 0,001 à 20 ppm.